# EUROPEAN PATENT APPLICATION

(11) **EP 1 429 274 A2**
(43) Date of publication of application: **16.06.2004**
(21) Application number: 03027469.0
(22) Date of filing: 01.12.2003
(51) Int. Cl.: G06F 19/00

(54) **Methods for sequence display and homology search**

(30) Priority: 10.12.2002 JP 2002358407
(71) Applicant: NEC CORPORATION, Tokyo (JP)
(72) Inventor: Miyakawa, Tomoya, Minato-ku, Tokyo (JP); Nakazato, Takeru, Minato-ku, Tokyo (JP); Asogawa, Minoru, Minato-ku, Tokyo (JP); Kenmochi, Akihisa, Minato-ku, Tokyo (JP)
(74) Representative: VOSSIUS & PARTNER

(57) **Abstract**

The method of displaying sequences of the present invention adds visual characteristics in accordance with the mutations and/or similarities in a plurality of similar nucleotide sequences or amino acid sequences. These visual characteristics are added to regions of mutation and/or regions of similarity, and further, are added in accordance with the degree of mutation and/or similarity and in accordance with the frequency of mutation in the regions of mutation. In addition, nucleotide sequences are converted to amino acid sequences, and the visual characteristics are added based on amino acid information that includes the names or properties of amino acids that correspond to codons of the regions of mutation. Links are provided to information that relates to a plurality of similar nucleotide sequences or amino acid sequences. The homology search method of the present invention uses this sequence display method to display search results.

## Description

The present invention relates to searching for homology of nucleotide sequences or amino acid sequences.

The human genome sequences (all human DNA sequences) have been substantially decoded, and databases (DB) for storing DNA sequences and the amino acid sequences that originate from DNA sequences have been built by such organizations as the NCBI (National Center for Biotechnology Information) of the NLM (National Library of Medicine), one facility belonging to the NIH (National Institutes of Health) in the United States, the National Institute of Genetics and the University of Tokyo Medical Research Institute in Japan, and the EBI (European Bioinformatics Institute) and EMBL (European Molecular Biology Laboratory) in Europe.

The genome sequences for other species of organisms besides humans such as mice, rats, blowfish, zebra fish, drosophilae, and nematodes are now either in the process of being decoded or have been decoded.

In addition, as the next stage, work is now in progress to investigate the differences in genome sequences between individual humans.

These circumstances underline the extreme importance of conducting homological comparisons between already known sequences and the nucleotide sequences or amino acid sequences that are being found in the course of research in the fields of biology and medicine. For example, if in the course of research a researcher obtains a sequence having extremely high homology with a nucleotide sequence or amino acid sequence that has already been registered by another researcher, the biological functions that originate in that sequence have, to some extent, already been analyzed, and the researcher must accordingly alter the orientation of his or her research. On the other hand, if a sequence having extremely high homology with a nucleotide sequence or amino acid sequence that has already been registered by another researcher has not yet been registered, the biological functions that originate in a nucleotide sequence or amino acid sequence that has been obtained in the course of research can be predicted based on already known sequences.

BLAST (Basic Local Alignment Search Tool, http:// www. ncbi. nlm. nih. gov/ blast/ ) is a system for homology searches of nucleotide sequences and amino acid sequences that has been developed by NCBI ( for example, refer to the Journal of Molecular Biology, 215, pp. 403∼410 (1990)). Through the use of BLAST, sequences having a high degree of homology can be obtained from, for example, Entrez ( http:// www. ncbi. nlm. nih. gov/ entrez/ ), which is a DB on nucleotide sequences and amino acid sequences provided by NCBI. This database is thus being used on a daily basis by biological and medical researchers around the world.

In addition to BLAST, FASTA (Fast Alignment) is a system for homology searches of nucleotide sequences and amino acid sequences (for example, refer to: Doolittle (Ed.), Methods in Enzymology, Academic Press, 183, pp. 63-98 (1990) ).

Sequence databases other than the NCBI database include the DDBJ (DNA Data Bank of Japan) that is managed by Japan's National Institute of Genetics and the nucleotide sequence and amino acid sequence database that is managed by EBI/EMBL. In addition, various nucleotide sequence and amino acid databases have been built for different purposes according to the aims of different organizations, one example being the JSNPs, which is a database of Single Nucleotide Polymorphisms of the human genome of the University of Tokyo Medical Research Institute in Japan, and these databases are used according to the purposes of each researcher.

When conducting a homology search of a nucleotide sequence or amino acid sequence in any of the above-described sequence databases, researchers generally use the above-described BLAST or FASTA system.

In addition, a sequence analysis that employs a homology search often necessitates a function for aligning corresponding parts of three or more nucleotide sequences or amino acid sequences based on homology (hereinbelow referred to as multiple sequence comparison or multiple alignment). Software for executing multiple alignment includes, for example, Clustal W and Clustal X that have been developed by EBI/EMBL. Many other types of multiple sequence comparison software are also available and being used by researchers, including software developed in Japan.

In a homology search of a nucleotide sequence or amino acid sequence, a query which is submitted to a sequence database is a nucleotide sequence or amino acid sequence that is supplied as input by the user or a nucleotide sequence or amino acid sequence that is obtained based on information that is supplied as input from the user, and similar sequences are searched. The points having the most significance in the search results are the points of dissimilarity of a nucleotide or amino acid (hereinbelow referred to as mutation) and points that are the same (hereinbelow referred to as similarity) between the nucleotide sequence or amino acid sequence that has been submitted in a query and the nucleotide sequence or amino acid sequence of the search results in those regions that are placed in correspondence by homology.

When using the BLAST or FASTA systems, several problems are encountered in obtaining information on points of mutation and points of similarity:
1. Since sequences that are submitted in queries and sequences that are searched are displayed in pairs, the user must cross-reference the comparison results for all pairs in order to obtain significant information such as the frequency or regions of mutation between a large number of sequences.
2. Since the search results are partially displayed by cutting out only parts having a high degree of homology, it is extremely difficult to comprehend which part is being displayed in relation to the entire sequence.
3. When nucleotides or amino acids for which parts are compared are the same, "|" or "." is shown, and the results are therefore difficult to comprehend at a glance.
4. Since the search results are displayed only in a simple text format, points of mutation can be easily overlooked.

To summarize the above-described points, the problem with the display of search results in the BLAST or FASTA system is the difficulty of accessing the important information that the user most requires such as the regions of mutation or regions of similarity between sequences.

As a countermeasure to the above-described problem, researchers often carry out a multiple alignment. Nevertheless, the above-described problem cannot be adequately overcome when using the above-described software for carrying out a multiple alignment.

As an example, the above-described Clustal X has a function for the color display of regions of mutation of amino acids. However, the standards for the coloration are not fixed and the coloration has no significance other than emphasizing mutation.

In addition, depending on the software that is used, the method of determining homology differs from the methods of BLAST or FASTA. Thus, when a multiple alignment is carried out with respect to search results of BLAST or FASTA, the corresponding nucleotide positions often deviate. In such a case, a procedure is then necessary for aligning the corresponding nucleotide positions using the search results that have been obtained by BLAST as a standard.

Further, when seeking homology through the use of this software, all sequences that are to be compared must be collected in advance. If a large number of sequences are to be compared, the procedure for verifying that no sequences are left out therefore places a heavy burden on the researcher.

It is therefore an object of the present invention to provide a sequence display method and homology search method that facilitate access to information relating to the regions of mutation and the regions of similarity between sequences, and moreover that enable display of all sequences that have been searched.

To achieve the above-described object, the sequence display method of the present invention adds visual characteristics according to the mutation and/or similarity in a plurality of similar nucleotide sequences or amino acid sequences.

The visual characteristics are added to regions of mutation and/or regions of similarity in a plurality of similar nucleotide sequences or amino acid sequences.

Further, the visual characteristics are added according to the degree of mutation and/or similarity in a plurality of similar nucleotide sequences or amino acid sequences.

Still further, the visual characteristics are added according to the frequency of the mutation in the regions of mutation of the plurality of similar nucleotide sequences or amino acid sequences.

By means of the above-described aspects, a user is able to immediately obtain important information such as mutations and similarities without the user himself or herself having to perform cross-referencing.

In addition, the visual characteristics are added based on amino acid information that includes the names and properties of amino acids that correspond to codons of the regions of mutation in the nucleotide sequence when the nucleotide sequences are converted to amino acid sequences. It is thus possible to evaluate the effect of mutation of a nucleotide on the generation of an amino acid.

These visual characteristics are represented by means of display colors, variation of display colors, or the variety of characters.

In addition, a plurality of similar nucleotide sequences or amino acid sequences are displayed with the corresponding nucleotides or amino acids in each sequence aligned. A user can thus easily comprehend the region, degree, and frequency relating to mutations or similarities of nucleotide or amino acid in the results of a multiple alignment.

In addition, links are provided to information that relates to a plurality of similar nucleotide sequences or amino acid sequences. Display information can thus include links between items of information that are included in homology search results or links between information that is included in homology search results and outside information such as information on the Internet, and this provision of linking information facilitates cross-referencing or viewing of reference information.

The homology search method of the present invention includes steps of: analyzing a query to a database of biological information that has been supplied as input by a user; based on the analysis of the query, generating search conditions that are appropriate for the database of biological information and searching the database of biological information; analyzing the search results; and displaying the search results using the analysis results and the above-described sequence display method. By means of this method, the steps from search to display are performed as a unit. As a result, even when carrying out a multiple alignment, the user is relieved of the burden of verifying whether some of the collected sequences have been missed.

In addition, a control unit may be provided in the homology search device for controlling the processes of each of the above-described steps.

The present invention as described hereinabove has the following effects:
First, information that has special significance such as the frequency and regions of mutation in the results of a homology search of nucleotide sequences and amino acid sequences can be obtained without requiring the user to cross-reference or reexamine. This effect can be obtained because homology search results that include a plurality of similar nucleotide sequences or amino acid sequences can be supplied as input and then supplied as display information to which visual characteristics have been added in accordance with the mutations and similarities in a plurality of similar nucleotide sequences or amino acid sequences.
Second, the regions of mutation in the plurality of sequences that are the results of a homology search can be comprehended at a glance. This effect can be obtained because display information can be supplied as output that includes visual characteristics that relate to regions of mutation in a plurality of similar nucleotide sequences or amino acid sequences.
Third, regions of similarity in a plurality of sequences that are the results of a homology search can be comprehended at a glance. This effect can be obtained because display information can be supplied as output that includes visual characteristics that relate to the regions of similarity in the plurality of similar nucleotide sequences or amino acid sequences.
Fourth, regions having a high degree of homology can be displayed together with the entirety of the plurality of sequences that are the results of a homology search. This effect can be obtained because the displayed information of the present invention can include the entirety of the sequences.
Fifth, results can be displayed while eliminating deviation of the positions of nucleotides in the results of a multiple alignment. This effect can be obtained because the displayed information can be supplied in a format that is aligned according to the positional correlation of the nucleotides and amino acids.
Sixth, sequence information that is to be compared can be collected in advance, or, when desired sequence information is not at hand, the sequence information can be acquired by using information that relates to sequences such as the name of a gene, amino acid, disease, or protein. This effect can be obtained because means are included for transmitting search condition information to a plurality of sequence databases.
Seventh, display is possible that allows immediate comprehension of regions of mutation and regions of similarity in sequences that are the result of homology searches and the results of multiple alignment. This effect can be obtained because the present invention can combine the above-described first to sixth effects.
Eighth, the operations that have to be performed by a user in the course of obtaining homology search results can be reduced, and the burden placed on the user can therefore be reduced. This effect can be obtained because positioning is not necessary in a multiple alignment, because links can be used to include cross-referencing in the display information, and because information relating to sequences can be used to obtain sequence information.
Ninth, the researcher need not alter the conditions and repeat the search when search conditions are altered to perform repeated searches, whereby an efficient homology search can be performed. This effect can be obtained because a control unit is included for controlling the series of operations for a search. The inclusion of this type of control unit enables control of the timing for submitting a query to a public sequence database, whereby the invention is also effective for reducing the load on a public sequence database server.

The above and other objects, features, and advantages of the present invention will become apparent from the following description with reference to the accompanying drawings, which illustrate examples of the present invention.
FIG. 1 shows the block diagram of a homology search device of the first embodiment of the present invention;
FIG. 2 is a flow chart showing the flow of processes in query analysis unit 11;
FIG. 3 is a flow chart showing the flow of processes in communication unit 12;
FIG. 4 is a flow chart showing the flow of processes in result analysis unit 13;
FIG. 5 is a flow chart showing the flow of processes in display unit 14;
FIG. 6 shows an example of the input screen in a homology search of a nucleotide sequence;
FIG. 7 shows a portion of an example of a result screen in a homology search of a nucleotide sequence;
FIG. 8 shows another portion of an example of the result screen in a homology search of a nucleotide sequence;
FIG. 9 shows an example of the input screen in a homology search of an amino acid sequence;
FIG. 10 shows an example of a result screen in a homology search of an amino acid sequence;
FIG. 11 shows amino acid transformation matrix BLOSUM 62;
FIG. 12 shows an example of a BLAST search result of the prior art; and
FIG. 13 shows the construction of the homology search device of the second embodiment of the present invention.

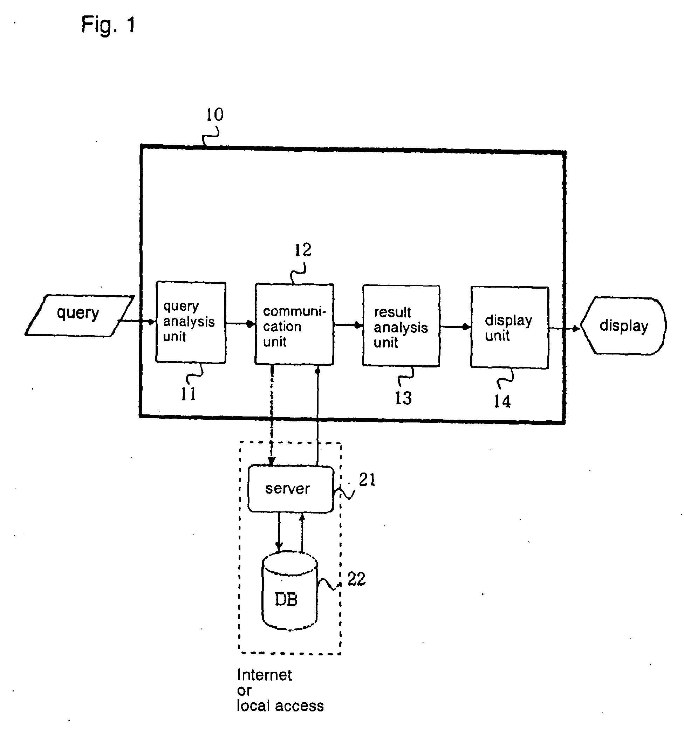

Referring now to FIG. 1, homology search device 10 according to the first embodiment of the present invention includes query analysis unit 11, communication unit 12, result analysis unit 13, and display unit 14.

Query analysis unit 11 accepts queries to sequence database 22 that have been supplied as input by a user, carries out an analysis, and supplies search conditions of the sequence as output to communication unit 12. A query that is received as input includes a nucleotide sequence or amino acid sequence, or an accession number, experimental data, or file as main information. A query further includes the designation of sequence database 22, biological species for restricting the results, whether to translate to an amino acid in the case of a nucleotide sequence, and whether to use PSI-BLAST (Position Specific Iterative Basic Local Alignment Search Tool) in the case of an amino acid sequence.

Communication unit 12 accepts the search conditions from query analysis unit 11 and transmits the search conditions to sequence database 22 by way of server 21. Communication unit 12 then receives the search results from sequence database 22 by way of server 21 and supplies the search results and search conditions as output to result analysis unit 13.

Result analysis unit 13 receives the search conditions and search results from communication unit 12 and performs an analysis of the search results. The analysis of search results includes the generation of information such as the regions, the frequency, and the degree of mutation. Result analysis unit 13 then supplies these results of analysis to display unit 14.

Display unit 14 receives the results of analysis from result analysis unit 13, adds visual information (for example, altering the color or the varieties of characters) based on the information such as the regions, frequency, and degree of mutation that is included in the analysis results, and further, generates and supplies display information.

Next, the operation of homology search device 10 is explained in detail with reference to the accompanying figures.

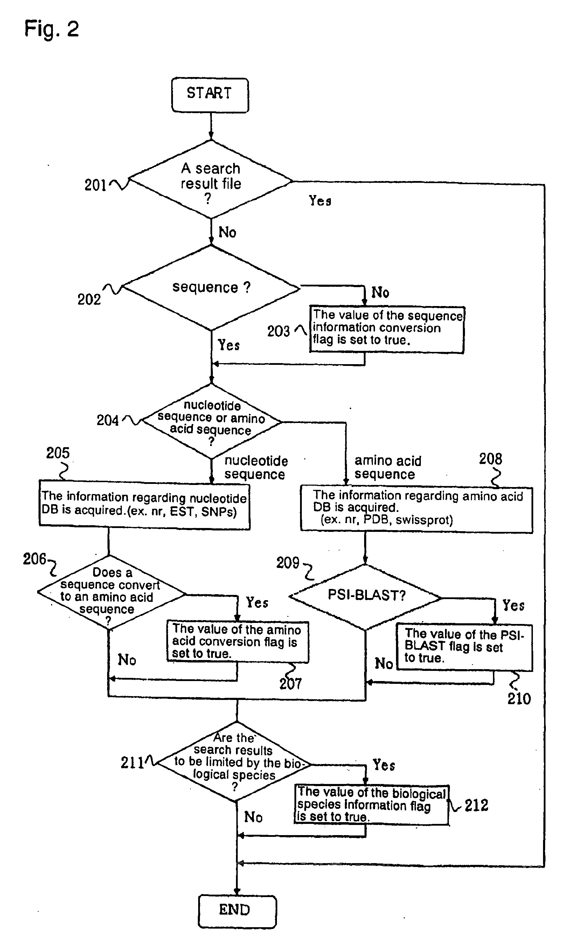

Referring first to FIG. 2, a flow chart is shown that illustrates the flow of processes in query analysis unit 11.

In Step 201, it is first determined whether or not a homology search is to be carried out based on a query that has been received as input. When the query is a sequence or an accession number, a flag is set such that a homology search is carried out. When the query designates a search result file, a flag is set such that a homology search is not carried out.

In Step 202, a flag is set indicating whether to convert from information that is included in the query to a sequence based on the query that has been received as input. For example, if the query includes an accession number, the value of the sequence information conversion flag is set to true in Step 203.

In Step 204, it is determined based on a query that has been received as input whether the received sequence or the sequence that has been indicated by received information is a nucleotide sequence or an amino acid sequence.

If it is determined that the sequence is a nucleotide sequence in Step 204, information is acquired in Step 205 regarding the database that the user has designated as the nucleotide sequence database. Nucleotide sequence databases include, for example, nr (Non-Redundant, a non-redundant sequence database that is a standard), EST (Expressed Sequence Tag, the sequence database of expressed genes), and SNPs (Single Nucleotide Polymorphisms, a single nucleotide polymorphism sequence database).

In Step 206, it is determined, based on the query that has been received as input, whether the received nucleotide sequence or a nucleotide sequence that is indicated by the received information is to be converted to an amino acid sequence and a further analysis to be carried out. If the sequence is to be converted, the value of the amino acid conversion flag is set to true in Step 207.

If it is determined in Step 204 that the received sequence or the sequence indicated by received information is an amino acid sequence, information is acquired in Step 208 regarding the amino acid sequence database that the user has designated. Amino acid sequence databases include, for example, nr (non-Redundant, a non-redundant sequence database that is the standard) PDB (Protein Data Bank, a database of the three-dimensional structures of proteins), swissprot (an amino acid sequence database) patent (a database of sequences in patents), yeast (a database of yeast sequences), and month (a database of sequences that have been recently added).

In Step 209, it is determined based on the query that has been received as input whether a PSI-BLAST (Position Specific Iterative-Basic Local Alignment Search Tool) is to be carried out. If PSI-BLAST is to be carried out, the value of the PSI-BLAST flag is set to true in Step 210.

In Step 211, it is determined based on the query that has been received as input whether the search results are to be limited by the biological species. Biological species here refers to, for example, a virus, bacteria, fungi, eukaryote, mammal, rodent, Arabidopsis thaliana, Bacillus subtilis, nematode, zebra fish, drosophila, colon bacillus, human, mouse, rat, or African clawed frog. When the user designates a limitation, the value of the biological species information flag is set to true in Step 212.

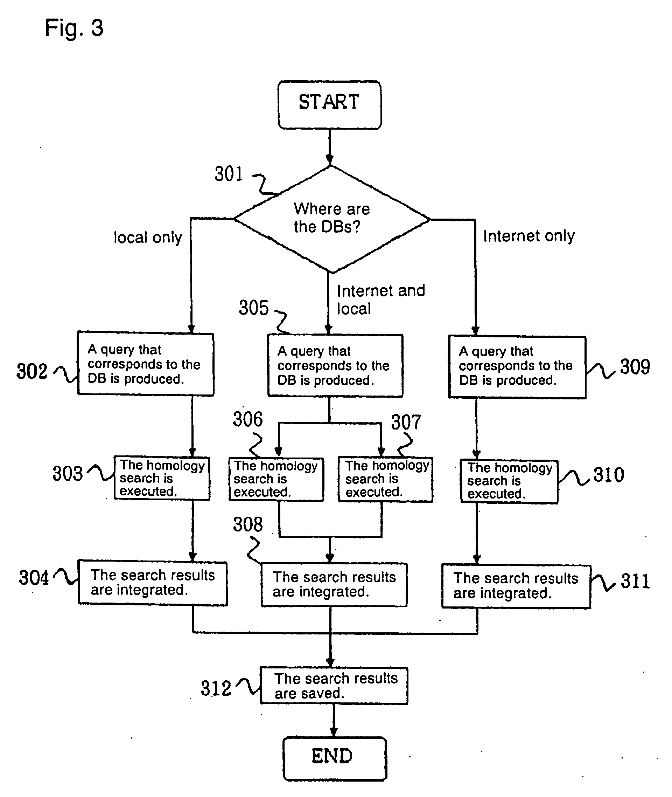

Referring now to FIG. 3, a flow chart is shown that illustrates the flow of processes in communication unit 12.

In Step 301, the location of the sequence database that was designated in Step 205 or Step 208 is determined.

In very general terms, the location of the sequence database can be divided between only local, both local and on the Internet, and only on the Internet. In this case, a local location indicates a range that includes homology search device 10 itself and a LAN (Local Area Network). The Internet indicates a range of a WAN (Wide Area Network, which employs connections using, for example, telephone lines).

If the location is limited to local, a query that corresponds to the sequence database is produced in Step 302 from the search conditions that were received from query analysis unit 11.

In Step 303, the query that was produced in Step 302 is passed on to the local server and the homology search is executed.

In Step 304, the results of the homology search that has been executed in Step 303 are integrated.

When the location is both on the Internet and local, a query is produced in Step 305 that corresponds to the sequence database based on the search conditions that were received from query analysis unit 11.

In Step 306, the query that was produced in Step 305 is turned over to the local server and a homology search is executed.

In Step 307, the query that was produced in Step 305 is transferred to the server on the Internet and the homology search is executed.

In Step 308, the results of the homology search that was executed in Steps 306 and 307 are integrated.

When the location is only the Internet, a query is produced in Step 309 that corresponds to the sequence database based on the search conditions that were received from query analysis unit 11.

In Step 310, the query that was produced in Step 309 is transferred to the server on the Internet and the homology search is executed.

In Step 311, the results of the homology search that was executed in Step 310 are integrated.

In Step 312, the search results that were integrated in Step 304, Step 308, or Step 311 are saved.

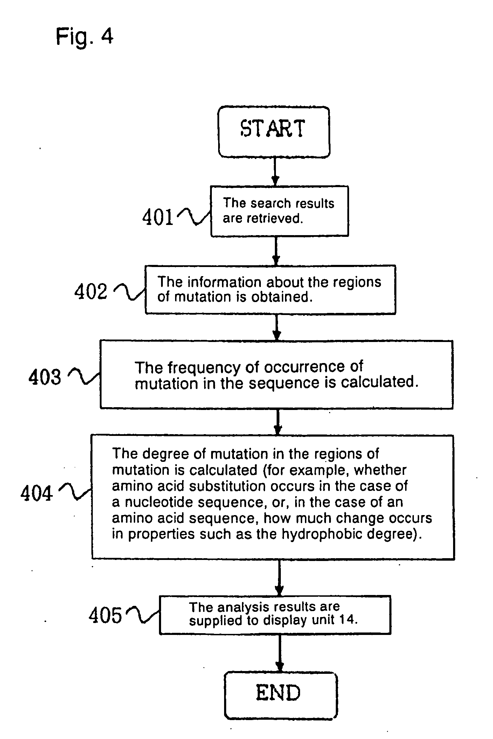

Referring now to FIG. 4, a flow chart is shown that illustrates the flow of processes in result analysis unit 13.

In Step 401, the search results that were received from communication unit 12 are fetched. When a homology search is not carried out, the file that is designated by the user is retrieved.

In Step 402, the search results that were fetched in Step 401 are analyzed, the regions of mutation of the sequence that was the subject of the query are identified, and information of these regions of mutation is obtained.

In Step 403, the information on the points of mutation that was obtained in Step 402 is integrated and the frequency of occurrence of this mutation in the sequences under comparison is calculated.

In Step 404, the degree of mutation in the regions of mutation that were obtained in Step 403 is calculated. If the sequences that are the subject of the query are nucleotide sequences and if a flag has been set in Step 207 in FIG. 2 for translating the nucleotide sequences to amino acid sequences and analyzing, the nucleotide sequences that have been received as input and the nucleotide sequences that have been searched are each converted to amino acid sequence and these amino acid sequences are then compared.

The degree of mutation described here refers to, for example, whether amino acid substitution occurs in the case of a nucleotide sequence, or, in the case of an amino acid sequence, how much change occurs in properties such as the hydrophobic degree or degree of acidity/basicity. In the case of an amino acid sequence or a nucleotide sequence that is translated to an amino acid sequence, the proximity of this property can be calculated by means of, for example, a transformation matrix known as BLOSUM 62. However, these are only examples, and calculation in terms of, not limited to, the acidity, hydrophilic degree, and/or isoelectric points, can be made.

In Step 405, the analysis results are supplied to display unit 14.

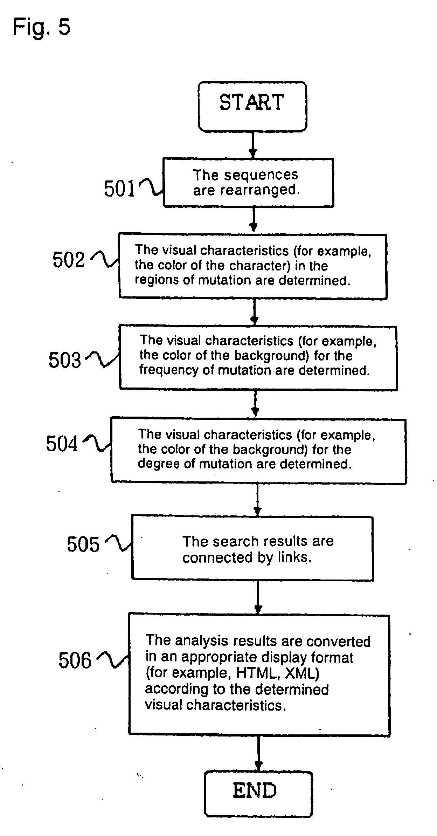

Referring now to FIG. 5, a flow chart is shown that illustrates the flow of processes in display unit 14.

In Step 501, the sequences are rearranged so that corresponding regions are aligned based on the information on the regions of mutation that has been analyzed in Step 402 in FIG. 4.

In Step 502, visual characteristics (such as the color of text or the type of text) of the regions of mutation are determined based on information on the regions of mutation that have been analyzed by Step 402 in FIG. 4.

The visual characteristics referred to here include; for example, the font, capital letter or small letter, and color of the text; and the color, pattern, texture, and animation of the background. However, these are only examples, and the visual characteristics may also include, for example, the size, thickness, underlining, italicization, flashing, shading, outlining and bordering of the text.

In Step 503, the visual characteristics (such as the color of the background) for the frequency of mutation are determined based on information regarding the frequency of mutation that was analyzed in Step 403 of FIG. 4. Here, frequency is preferably represented using variation in the display color. This variation in the display color means, for example, variation of the shade or the hue of the color (as one example, variation from red to blue).

In Step 504, the visual characteristics (for example, the background color) for the degree of mutation are determined based on the information regarding the degree of mutation that was analyzed in Step 404 of FIG. 4. Here, the degree is preferably represented using variation of the display color. This variation in the display color means, for example, variation of the shade or the hue of the color (as one example, variation from red to blue).

In Step 505, information that is included in the results of the homology search is connected by links.

In Step 506, conversion is executed to an appropriate display format according to the output and based on information of the visual characteristics that were applied by Steps 501 to 505 of FIG. 5. The output referred to here may be, for example, output for a screen or printer, and the display format may be, for example, HTML (Hyper Text Markup Language) or XML (extensible Markup Language). However, these are only examples, and the display format may also be TeX, bmp, gif, jpeg, PNG, TIFF, PICT, PDF (Portable Document Format), or PostScript.

An example of the operation of the homology search device of the first embodiment is next described in detail with reference to the accompanying figures. In the following description, each of the steps that are shown in the flow charts of FIG. 2 to FIG. 5 has been converted to computer program form, this computer program being recorded on a recording medium and a personal computer that serves as homology search device 10 being caused to read this recording medium and operate accordingly.

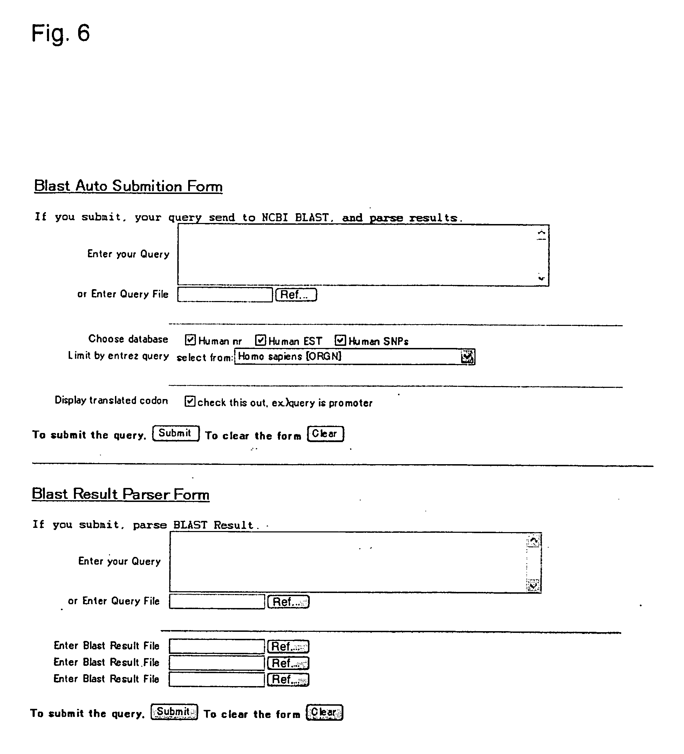

Referring now to FIG. 6, an input screen is shown for conducting a homology search for a nucleotide sequence. Here, the user carries out: input of the nucleotide sequence (Enter your Query), designation of the sequence database in which the search is to be performed (Choose database), designation of whether the nucleotide sequence is to be translated to an amino acid and analyzed (Display translated codon), designation of the biological species for limiting the search results (Limit by entrez query), and designation of a search result file that has not yet been analyzed (Enter Query File).

After the user has supplied the nucleotide sequence as input in the nucleotide sequence input form (Enter your Query), the user clicks on the Submit button, whereupon query analysis unit 11 receives the nucleotide sequence. Each unit then executes each of the processes as previously described.

In the present example, the user is able to designate whether the nucleotide sequences are to be translated to amino acid sequences and analyzed. This designation is saved as the amino acid conversion flag. If the flag is true, the nucleotide sequence that has been received as input is converted to an amino acid sequence, and the degree of mutation is calculated by considering the amino acid sequence. In this way, the user can easily understand whether the mutation of the nucleotide accompanies amino acid substitution, and can obtain an indication as to whether this mutation must be restored to its origin by experimental manipulation.

In the present example, moreover, the user is able to designate the sequence database that is to be searched. Queries can therefore be distributed based on the information of sequence databases that has already been saved. For example, if the user designates the nucleotide sequence that is the subject of a query and nr and EST as the sequence databases, the homology search is carried out for this nucleotide sequence in each of nr and EST. Thus, the user is not only able to inclusively obtain necessary information by designating a number of sequence databases, but is also able to exclude redundant search results by selecting sequence databases that are consistent with the purpose of the search, and thus obtain search results that can be easily understood.

In the present example, moreover, the user is able to designate whether the search results are to be limited by biological species. This designation is held in the biological species information flag. If the flag is true, only the results of comparison with sequences of the designated biological species are displayed. For example, the user can designate human, mouse, and rat as the biological species, whereby the user can exclude redundant search results and thus obtain search results that can be more easily understood.

In addition, in the present example, the user is able to select, as the object of analysis, results that have already been obtained in a past homology search. For example, a file for which results have not yet been analyzed can be designated. In such a case, a flag is set such that only analysis of results is performed without conducting a homology search. As a result, the user can take advantage of an analysis used in the present invention to better understand search results that have not been derived by the present invention.

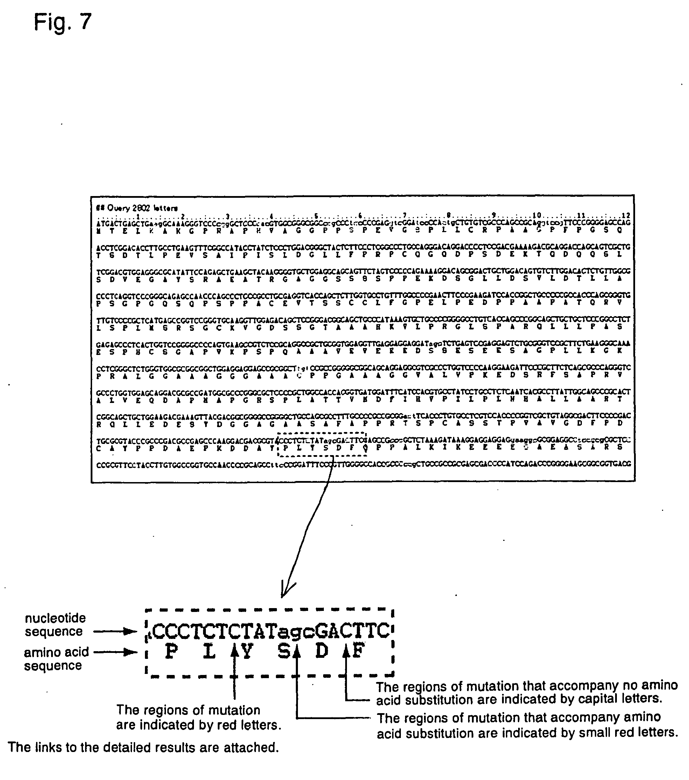

Referring now to FIG. 7, a portion is shown of the results screen when carrying out a homology search for a nucleotide sequence. FIG. 7 shows a nucleotide sequence for which a query was submitted. The regions of mutation of the nucleotide sequence for which a query was submitted are indicated by red letters. The corresponding amino acid sequence is described below the nucleotide sequence. In particular, the regions of mutation of the nucleotide sequence that accompanies amino acid substitution are indicated by small letters. In this way, the user can easily understand whether the difference of the nucleotide accompanies amino acid substitution and can judge whether this difference must be restored to the original state by an experimental operation.

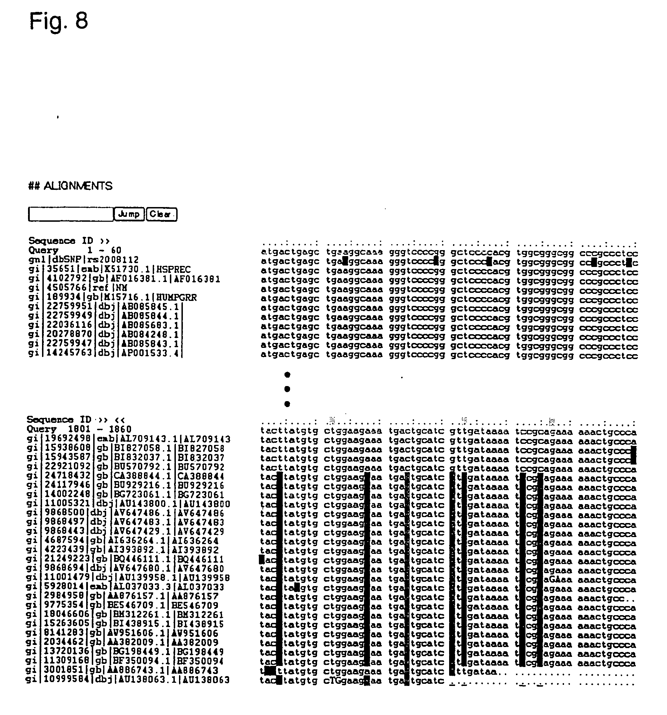

In addition, links to the detailed results of a multiple alignment comparison (to be explained in relation to FIG. 8) are attached to each of the regions of mutation, whereby the user can cross-reference both the overall state of difference of sequences for which a query was submitted as well as the details of each difference.

Referring now to FIG. 8, another portion is shown of the results screen when a homology search is performed for a nucleotide sequence. FIG. 8 is the results screen for a multiple alignment comparison. The sequence in the upper level is the sequence for which a query was submitted that was shown in FIG. 7, and the sequence below is a sequence that results from the homology search. The sequences are divided into groups of 60 characters. The number of characters may be, for example, 90 characters, 120 characters, or the greatest number of characters that can be displayed. In the present example, the translation of three nucleotides to correspond to one amino acid is taken into consideration, and characters are therefore divided into groups of a number that is divisible by 3, and moreover, for the sake of facilitating the understanding of the user, a number that is divisible by 10, i.e., a multiple of 30.

In a region of mutation, and in particular, in a region in which the insertion of a nucleotide is seen, the background color is made yellow to add special emphasis. This emphasis indicates the insertion or omission of a nucleotide and means that the amino acid sequence changes completely from this region on, this being particularly significant information for the researcher.

Further, in each region of mutation, the shade of the background color indicates the frequency of occurrence of the mutation. The user can therefore get an indication of whether this mutation is a nucleotide sequence substitution that cannot be avoided in experimentation, is a characteristic peculiar to the sequence (for example, when the mutation is the cause of a particular disease), or must be restored to its original state by experimentation.

In addition, sequences are displayed by aligning corresponding parts based on homology.

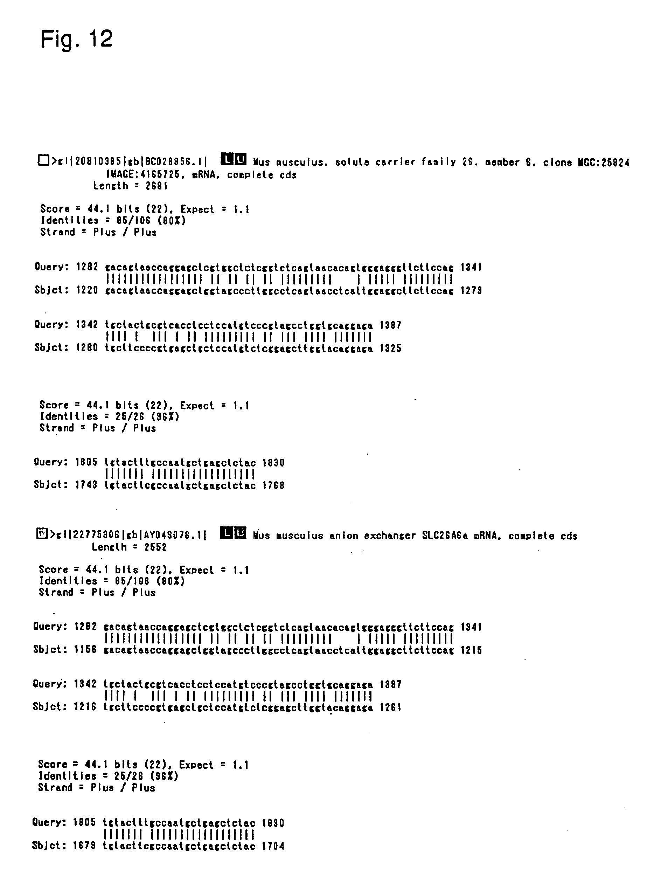

Further, information is attached for linking to the already existing sequence comparison results of FIG. 12. The user can, by designating which nucleotide of the sequences the user wishes to see, display the sequence comparison in that region. In this way, the user is able to inclusively cross-reference information on mutations.

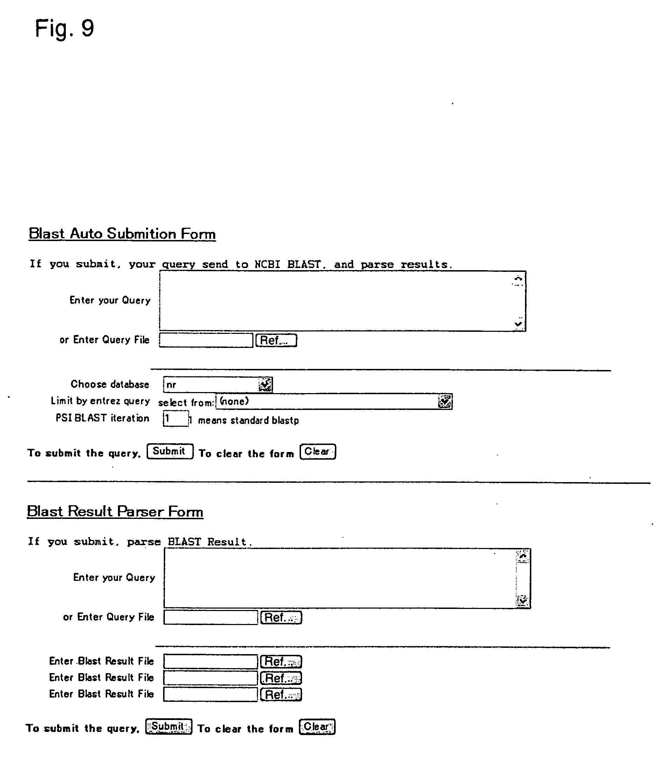

Referring now to FIG. 9, an input screen is shown for carrying out a homology search for amino acid sequences. Here, the user can perform: input of the amino acid sequence (Enter your Query), designation of the sequence database that is to be searched (Choose database), designate whether PSI-BLAST is to be performed (PSI-BLAST iteration), designation of the biological species for limiting the search results (Limit by entrez query), and designation of search result files that have not yet been analyzed (Enter Query File).

After the user has entered the amino acid sequence in the amino acid sequence input form (Enter your Query), the user clicks on the Submit button, and query analysis unit 11 accepts the amino acid sequence. Each of the units then executes processing as described hereinabove.

In the present example, the user is able to designate whether to obtain search results using PSI-BLAST. This designation is held as the PSI-BLAST flag. The user is therefore able to use PSI-BLAST as a means for obtaining the search results, and is able to perform comparison for sequences having homology that cannot be obtained by methods other than PSI-BLAST.

In addition, the user is also able to designate whether to limit the results by biological species. This designation is held in the biological species flag.

The user is further able to select results that have been obtained in a past homology search as the object of analysis.

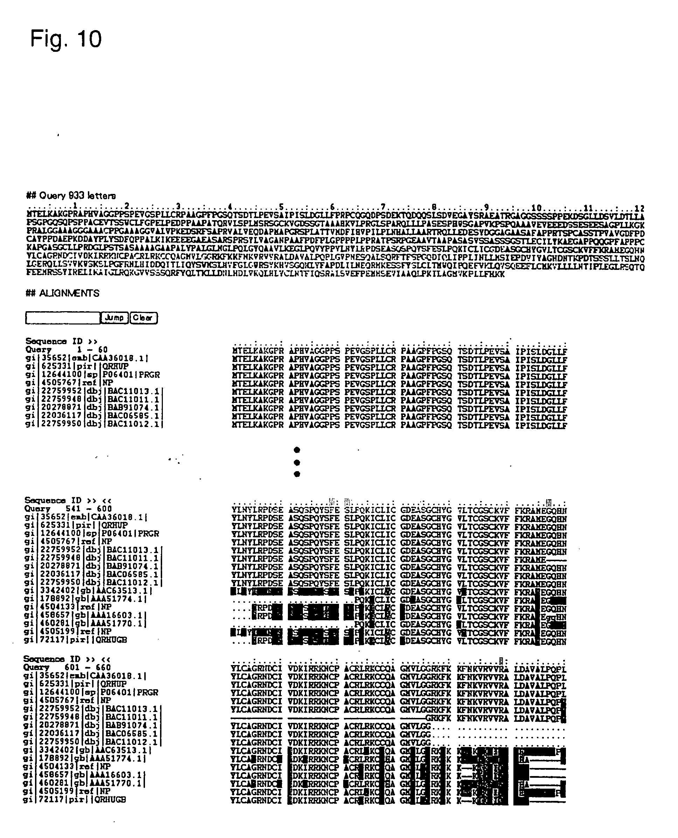

Referring to FIG. 10, a result screen is shown for a case in which a homology search is carried out for amino acid sequences. The upper portion of FIG. 10 is the amino acid sequence for which the query has been submitted, this amino acid sequence corresponding to FIG. 7 for a nucleotide sequence. The lower portion of FIG. 10 is the result of comparison of multiple alignments and corresponds to FIG. 8 for a nucleotide sequence.

Of the amino acid sequence that has been applied as input, the regions of mutation are indicated by red letters, whereby the user can easily comprehend the overall state of the regions of mutation for the sequence for which the query was submitted.

In addition, each of the sequences of a multiple alignment is divided into sections of 60 characters.

The sequences are displayed with corresponding portions aligned based on homology.

In each of these differences, the degree of difference is indicated by the shade of the background color.

In addition, the background color is highlighted yellow when an insertion of an amino acid is seen.

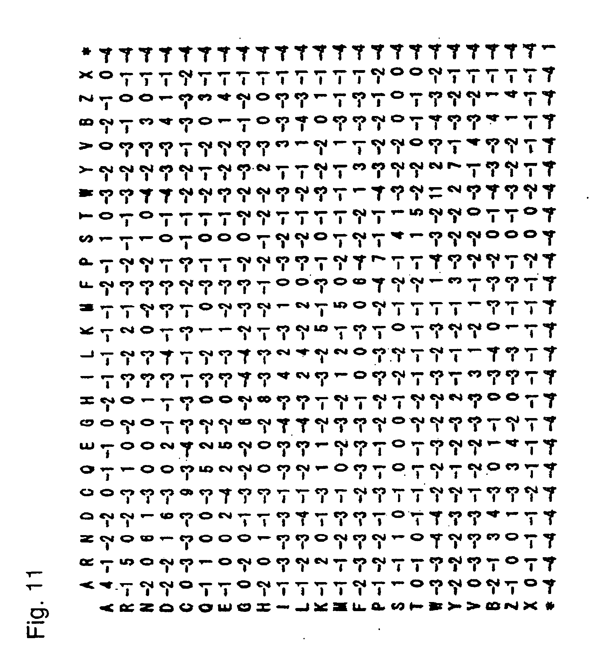

Twenty types of amino acid are used in organisms, and such properties as hydrophobicity or acidity/basicity have been determined for each. When these properties change together with the differences, the properties of the overall sequence can be predicted to change, and in addition, the functions of the enzyme are lost when the points of difference are the central positions of reactivity of the enzyme. Based on this fact, a transformation matrix that describes the differences in properties between amino acids is used to calculate the proximity of properties between amino acids, and this value is represented by varying the shade of the background color. In the present example, the transformation matrix BLOSUM 62 that is shown in FIG. 11 is used as this transformation matrix.

In FIG. 10, information is attached regarding links to the already existing sequence comparison results of FIG. 12. By designating which nucleotide of a sequence he or she wishes to see, the user can display the relevant region, whereby the user is able to inclusively cross-reference information on mutations.

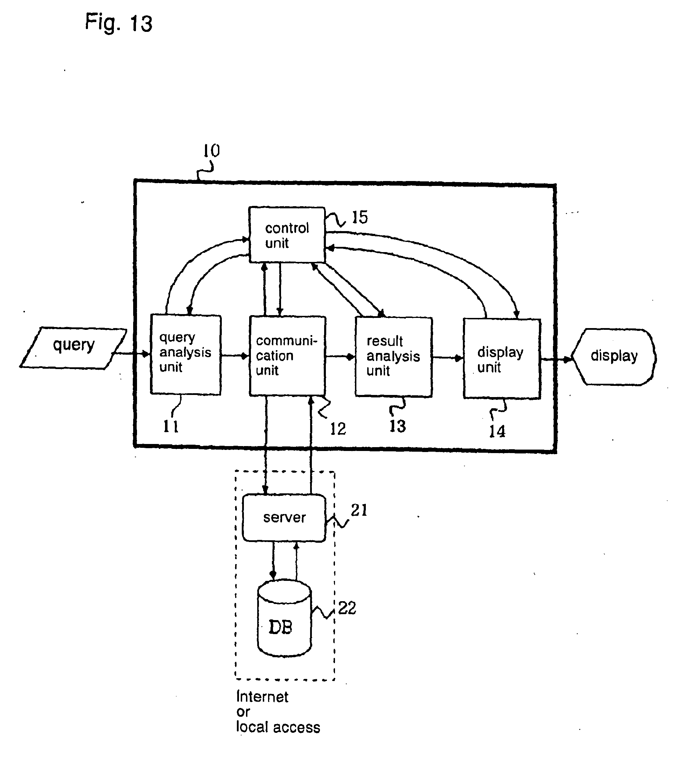

Referring to FIG. 13, the homology search device according to the second embodiment of the present invention differs from the homology search device of the first embodiment in that it includes control unit 15, and the following explanation is therefore limited to control unit 15.

Control unit 15 controls the operation of query analysis unit 11, communication unit 12, result analysis unit 13 and display unit 14. The provision of control unit 15 enables the control of the timing of processing in each unit by monitoring the generation of analysis results and monitoring the congested state in communication circuits when performing continuous processing of queries or when searching a plurality of sequence databases.

Control unit 15 receives notification of the processing state from query analysis unit 11, communication unit 12, result analysis unit 13, and display unit 14; and in accordance with these notifications, effects control such that processing of each unit is carried out in succession. As a result, query analysis unit 11, communication unit 12, result analysis unit 13, and display unit 14 of the second embodiment each further have the capability to report the completion of control to control unit 15 upon completing processing, and operate in accordance with operation control from control unit 15. As a result, the user need not enter each and every query when the user wishes to perform automatic search and analysis for a large number of queries, and the burden on the user is therefore greatly reduced.

While preferred embodiments of the present invention have been described using specific terms, such description is for illustrative purposes only, and it is to be understood that changes and variations may be made without departing from the spirit or scope of the following claims.

## Claims

1. A sequence display method, comprising:
a first step of accepting, by a sequence display device, information relating to mutations or similarities between a plurality of similar nucleotide sequences or amino acid sequences; and
a second step of adding visual characteristics according to said mutations or similarities and displaying said plurality of similar nucleotide sequences or amino acid sequences.

2. A sequence display method according to claim 1, wherein said
visual characteristics are added to regions of mutation and/or regions of similarity in said plurality of similar nucleotide sequences or amino acid sequences.

3. A method according to claim 1 or 2, wherein said
visual characteristics are added in accordance with the degree of said mutation and/or similarity.

4. A method according to claim 1 or 2, wherein said
visual characteristics are added in accordance with the frequency of mutation in said regions of mutation.

5. A method according to claim 1, 2, 3 or 4, wherein said
visual characteristics are added based on amino acid information that includes the names and properties of amino acids that correspond to codons in said plurality of similar nucleotide sequences.

6. A method according to any one of claims 1 to 5, wherein said
visual characteristics are displayed using display color.

7. A method according to any one of claims 1 to 5, wherein said
visual characteristics are displayed using variation of display color.

8. A method according to any one of claims 1 to 5, wherein said
visual characteristics are displayed using varieties of characters.

9. A method according to any one of claims 1 to 8, wherein, in
said second step, corresponding nucleotides or amino acids in each sequence are displayed in alignment.

10. A method according to any one of claims 1 to 9, further comprising a third step of adding links between said plurality of similar nucleotide sequences or amino acid sequences and/or links to relevant information.

11. A sequence display device, comprising:
a first means for accepting information relating to mutation and/or similarity in a plurality of similar nucleotide sequences or amino acid sequences; and
a second means for adding visual characteristics in accordance with said mutation and/or similarity and displaying said plurality of similar nucleotide sequences or amino acid sequences.

12. A sequence display device according to claim 11, wherein said
second means adds said visual characteristics to regions of mutation and/or regions of similarity in said plurality of similar nucleotide sequences or amino acid sequences.

13. A device according to claim 11 or 12, wherein said
second means adds said visual characteristics in accordance with the degree of said mutation and/or said similarity.

14. A device according to claims 11, 12 or 13, wherein said
second means adds said visual characteristics in accordance with the frequency of mutation in said regions of mutation.

15. A device according to claim 11, 12, 13 or 14, wherein said
second means adds said visual characteristics based on amino acid information that includes the names and properties of amino acids that correspond to codons in said plurality of similar nucleotide sequences.

16. A device according to any one of claims 11 to 15, wherein said
second means represents said visual characteristics using display color.

17. A device according to any one of claims 11 to 16, wherein said
second means represents said visual characteristics using variation of display color.

18. A device according to any one of claims 11 to 17, wherein said
second means represents said visual characteristics using varieties of characters.

19. A device according to any one of claims 11 to 18, wherein said
second means displays with corresponding nucleotides or amino acids in each sequence in alignment.

20. A device according to any one of claims 11 to 19, further
comprising third means for adding links between said plurality of similar nucleotide sequences or amino acid sequences and/or links to relevant information.

21. A sequence display program product for causing a computer to execute each of the steps described in any one of claims 1 to 10.

22. A recording medium on which is recorded a sequence display program for causing a computer to execute each of the steps described in any one of claims 1 to 10 and that can be read by a computer.

23. A homology search method, comprising:
a fourth step of analyzing a query to a sequence database that has been submitted by a user;
a fifth step of generating search conditions that are appropriate for said sequence database based on the analysis results of said fourth step and searching said sequence database;
a sixth step of analyzing the search results of said fifth step and generating information relating to mutations and/or similarities in a plurality of similar nucleotide sequences or amino acid sequences; and
a seventh step of using information that has been generated in said sixth step and a sequence display method described in any one of claims 1 to 10 to display search results of said fifth step.

24. A homology search device, comprising:
a fourth means for analyzing a query to a sequence database that has been submitted by a user;
a fifth means for generating search conditions that are appropriate for said sequence database based on analysis results produced by said fourth means and searching said sequence database;
a sixth means for analyzing search results produced by said fifth means and generating information relating to mutations and/or similarities in a plurality of similar nucleotide sequences or amino acid sequences; and
a seventh means for using information that has been generated by said sixth means and a sequence display device according to any one of claims 11 to 20 to display search results produced by said fifth means.

25. A homology search device according to claim 24, further comprising an eighth means for controlling the operation of the fourth means, the fifth means, the sixth means, and the seventh means.

26. A homology search program product for causing a computer to execute each of the steps described in claim 23, 24 or 25.

27. A recording medium on which is recorded a homology search program for causing a computer to execute each of the steps described in claim 23, 24 or 25 and that can be read by a computer.
